# EUROPEAN PATENT APPLICATION

(11) **EP 4 378 451 A1**
(43) Date of publication of application: **05.06.2024**
(21) Application number: 22848634.6
(22) Date of filing: 28.07.2022
(51) Int. Cl.: A61K 9/08, A61K 31/506, A61P 35/00

(54) **PAZOPANIB ORAL PHARMACEUTICAL COMPOSITION, AND PREPARATION METHOD THEREFOR AND USE THEREOF**

(30) Priority: 30.07.2021 CN 202110871277
(71) Applicant: Shanghai Aurora Biotechnology Co., Ltd., Shanghai 201210 (CN)
(72) Inventor: GUO, Zhen, Shanghai 201210 (CN); WANG, Xuan, Shanghai 201210 (CN); LU, Pengcheng, Shanghai 201210 (CN); FU, Jun, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2022/108602
(87) International publication number: WO 2023/006029

(57) **Abstract**

Provided are a pazopanib oral pharmaceutical composition, and a preparation method therefor and the use thereof. Provided is a pazopanib oral pharmaceutical composition, which comprises an active drug, a polymer carrier, an organic solvent and/or water, wherein the active drug is 5-[[4-[(2,3-dimethyl-2H-indazol-6-yl)methylamino]pyrimidin-2-yl]amino]-2-methylbenzenesulfonamide as represented by formula I and a salt thereof. Dispersion of pazopanib in the form of a solution can significantly improve the solubility and dissolution rate of pazopanib, can significantly increase the drug bioavailability, and can also improve the compliance of a patient and solve the problems of dysphagia in elderly patients, etc.

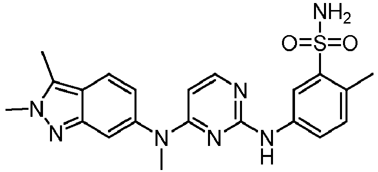

## Description

The present application claims the priority to Patent Application No. 202110871277.1, entitled "PAZOPANIB ORAL PHARMACEUTICAL COMPOSITION, AND PREPARATION METHOD THEREFOR AND USE THEREOF" filed with the China National Intellectual Property Administration on July 30, 2021, which is incorporated herein by reference in its entirety.

### TECHNICAL FIELD

The present disclosure relates to a pazopanib oral pharmaceutical composition, a preparation method therefor and use thereof.

### BACKGROUND

Renal cell carcinoma, renal cancer for short, is one of the most common tumors in the urinary system, with the morbidity accounting for 2% of adult malignant tumors. In China, the morbidity of renal cell carcinoma ranks second among urinary tumors, second only to bladder cancer. With the continuous progress of diagnostic technologies, renal cancer patients have been treated earlier. The overall 5-year survival rate for renal cancer reaches 74%, but the number of advanced metastasis patients is only 12%.

Vascular endothelial growth factor (VEGF) is an angiogenesis factor in the tumor angiogenesis process, and is a hormone regulator for endothelial cell differentiation. The development of solid tumors is closely related to the expression of VEGF, and VEGF is a key angiogenesis-promoting factor in tumor neovascularization, is combined with VEGF receptor tyrosine kinase specifically and highly expressed on the surface of neovascular endothelial cells, activates tyrosine kinase, and thus plays a biological function. Therefore, tumor vascular targeting therapy (which means using VEGF receptor tyrosine kinase inhibitors) targeting VEGF receptor tyrosine kinase has become a new approach for tumor therapy in recent years.

Pazopanib (5-[[4-[(2,3-dimethyl-2H-indazol-6-yl)methylamino]pyrimidin-2-yl]amino]-2-methylbenzenesulfonamide) is a novel oral angiogenesis inhibitor developed by GlaxoSmithKline that can interfere with neovascularization required for the survival and growth of refractory tumors, targets the vascular endothelial growth factor receptors (VEGFRs), and acts by inhibiting neovascularization of the blood supply to tumors. It is a drug that can treat a variety of tumors, and has shown positive results in large-scale clinical trials involving patients with soft tissue sarcoma and renal cell carcinoma.

Pazopanib is a BCS II drug, belonging to insoluble hypertonic drugs. The low solubility limits its bioavailability, and in order to enable pazopanib to be quickly dissolved out, improve the bioavailability and reduce side effects, the research on a pharmaceutical composition with immediate-release property has important social and economic values.

At present, a pazopanib formulation that has simple preparation process, easy scale-up production, more convenient dosage adjustment and good patient compliance is continuously developed.

### SUMMARY

The present disclosure provides a pazopanib oral pharmaceutical composition, which comprises an active drug, a polymeric carrier, an organic solvent and/or water, wherein the active drug is 5-[[4-[(2,3-dimethyl-2H-indazol-6-yl)methylamino]pyrimidin-2-yl]amino]-2-methylbenzenesulfonamide as represented by formula I or a salt thereof;

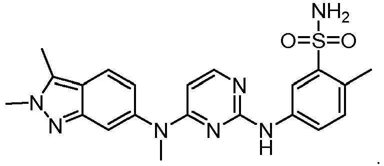

According to an embodiment of the present disclosure, the pazopanib oral pharmaceutical composition comprises: an active drug, a polymeric carrier, an organic solvent, and water, wherein the active drug is 5-[[4-[(2,3-dimethyl-2H-indazol-6-yl)methylamino]pyrimidin-2-yl]amino]-2-methylbenzenesulfonamide represented by formula I or a salt thereof; the pH value of the pazopanib oral pharmaceutical composition is 3.0-4.5;

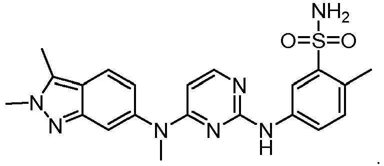

According to an embodiment of the present disclosure, the polymeric carrier is selected from one or more of polyvinyl caprolactam-polyvinyl acetate-polyethylene glycol graft copolymer (soluplus), poloxamer, polyethylene glycol vitamin E succinate (TPGS), 15-hydroxystearic acid polyethylene glycol ester, polyoxyethylene 40 hydrogenated castor oil, polyoxyl 35 hydrogenated castor oil, povidone, crospovidone, hydroxypropyl methylcellulose (HPMC), hydroxypropylcellulose, polyvinyl alcohol, tween 80, polyvinylpyrrolidone (PVP), and polyethylene glycol; as an example, the polymeric carrier is selected from a combination of soluplus and PVP, a combination of soluplus and TPGS, a combination of TPGS and HPMC, and soluplus.

According to an embodiment of the present disclosure, the weight ratio of the active drug to the polymeric carrier is 1:10-10:1, e.g., 3:10, 3:8, 3:5, 2:5, 10:1, 2:1, 1:2, 5:16, 1:4, or 1:8. According to an embodiment of the present disclosure, the active drug is preferably 5-[[4-[(2,3-dimethyl-2H-indazol-6-yl)methylamino]pyrimidin-2-yl]amino]-2-methylbenzenesulfonamide hydrochloride (pazopanib hydrochloride).

According to an embodiment of the present disclosure, the concentration of the active drug is 5-20 mg/mL, such as, 6-15 mg/mL, as an example, 7 mg/mL, 7.5 mg/mL, 8 mg/mL, 9 mg/mL, 10 mg/mL, 11 mg/mL, 12 mg/mL, 12.5 mg/mL, 13 mg/mL, or 14 mg/mL.

According to an embodiment of the present disclosure, the organic solvent is selected from one or more of ethanol, propylene glycol, glycerol, polyethylene glycol-300, polyethylene glycol-400, and polyethylene glycol-600; as an example, the organic solvent is selected from a combination of ethanol and glycerol, and a combination of ethanol, propylene glycol and glycerol.

According to an embodiment of the present disclosure, the volume ratio of the organic solvent to the pazopanib pharmaceutical composition is 0.3-0.9, and the volume ratio refers to the ratio of the volume of the organic solvent to the total volume of the pazopanib pharmaceutical composition; as an example, the volume ratio is 0.3, 0.4, 0.5, 0.6, 0.7, 0.75, 0.8, 0.85, or 0.9. According to the embodiment of the present disclosure, the volume ratio of the water to the pazopanib pharmaceutical composition is 0.1-0.7, and the volume ratio refers to the ratio of the volume of the water to the total volume of the pazopanib pharmaceutical composition; as an example, the volume ratio is 0.1, 0.15, 0.2, 0.25, or 0.3.

According to an embodiment of the present disclosure, the pazopanib oral pharmaceutical composition may further comprise a sweetener.

According to an embodiment of the present disclosure, the sweetener is selected from one or more of aspartame, sucralose, fructose, sucrose, stevioside, glycyrrhizin, essence, spices, saccharin and sodium saccharin.

According to an embodiment of the present disclosure, the mass ratio of the sweetener to the active drug is 1:5-5:1, such as 1:3, 1:4, or 2:1.

According to an exemplary embodiment of the present disclosure, the pazopanib oral pharmaceutical composition may be in any one of the following formulas:
Formula 1: 7.5 mg/mL pazopanib hydrochloride, 20 mg/mL soluplus, 5 mg/mL PVP, 10% v/v of ethanol, 20% v/v of glycerol, 2.5 mg/mL essence, and 70% v/v of water;
Formula 2: 7.5 mg/mL pazopanib hydrochloride, 10 mg/mL soluplus, 10 mg/mL TPGS, 20% v/v of ethanol, 5% v/v of propylene glycol, 25% v/v of glycerol, 2.5 mg/mL essence, and 50% v/v of water;
Formula 3: 7.5 mg/mL pazopanib hydrochloride, 10 mg/mL TPGS, 2.5 mg/mL HPMC, 15% v/v of ethanol, 15% v/v of propylene glycol, 30% v/v of glycerol, 2.5 mg/mL essence, and 40% v/v of water;
Formula 4: 10 mg/mL pazopanib hydrochloride, 20 mg/mL soluplus, 5 mg/mL PVP, 10% v/v of ethanol, 60% v/v of glycerol, 2.5 mg/mL essence, and 30% v/v of water;
Formula 5: 10 mg/mL pazopanib hydrochloride, 1 mg/mL soluplus, 20% v/v of ethanol, 15% v/v of propylene glycol, 40% v/v of glycerol, 5 mg/mL stevioside, and 25% v/v of water;
Formula 6: 10 mg/mL pazopanib hydrochloride, 5 mg/mL soluplus, 25% v/v of ethanol, 50% v/v of glycerol, 5 mg/mL stevioside, and 25% v/v of water;
Formula 7: 10 mg/mL pazopanib hydrochloride, 20 mg/mL soluplus, 20% v/v of ethanol, 15% v/v of propylene glycol, 50% v/v of glycerol, 5 mg/mL stevioside, and 15% v/v of water;
Formula 8: 12.5 mg/mL pazopanib hydrochloride, 40 mg/mL soluplus, 25% v/v of ethanol, 60% v/v of glycerol, and 15% v/v of water;
Formula 9: 12.5 mg/mL pazopanib hydrochloride, 50 mg/mL soluplus, 30% v/v of ethanol, 10% v/v of propylene glycol, 50% v/v of glycerol, and 10% v/v of water; and
Formula 10: 12.5 mg/mL pazopanib hydrochloride, 100 mg/mL soluplus, 25% v/v of ethanol, 15% v/v of propylene glycol, 50% v/v of glycerol, and 10% v/v of water.

The present disclosure further provides a method for preparing the pazopanib pharmaceutical composition, which comprises the following steps:
(1) mixing the polymeric carrier and the sweetener with water to form a solution A;
(2) mixing the organic solvent with the solution A obtained in the step (1) to obtain a solution B; and
(3) mixing the active drug with the solution B obtained in the step (2), stirring and filtering to obtain the pazopanib pharmaceutical composition.

According to an embodiment of the present disclosure, in the step 3, the filtration is filtration through a filter membrane, and the pore size of the filter membrane may be 0.01 µm to 1.0 µm, such as 0.45 µm.

The present disclosure further provides use of the pazopanib oral pharmaceutical composition for the manufacture of a medicament for treating and/or preventing a tumor.

According to an embodiment of the present disclosure, the tumor may be renal cancer or soft tissue sarcoma.

The present disclosure further provides use of the pazopanib oral pharmaceutical composition for manufacturing of a pharmaceutical formulation.

The present disclosure further provides a pharmaceutical formulation comprising the pazopanib oral pharmaceutical composition.

As an example, the pharmaceutical formulation may be an oral liquid.

The present disclosure further provides a method for treating and/or preventing a tumor, which comprises administering to populations in need thereof a therapeutically effective amount of the pazopanib oral pharmaceutical composition or the pharmaceutical formulation.

### Advantageous Effect

The present disclosure provides a pazopanib oral pharmaceutical composition, a preparation method therefor and use thereof, aiming at overcoming the defects of low solubility, low bioavailability, large side effect, poor patient compliance and the like of pazopanib in the prior art. Pazopanib in the composition or the formulation of the present disclosure is dispersed in a solution form, so the solubility and the dissolution speed of pazopanib can be significantly improved, the bioavailability of the drug is significantly improved, and pazopanib has the advantages of high solubility, high dissolution speed and high bioavailability, and can both improve patient compliance and solve the problems of dysphagia in elderly patients and the like.

### BRIEF DESCRIPTION OF THE DRAWING

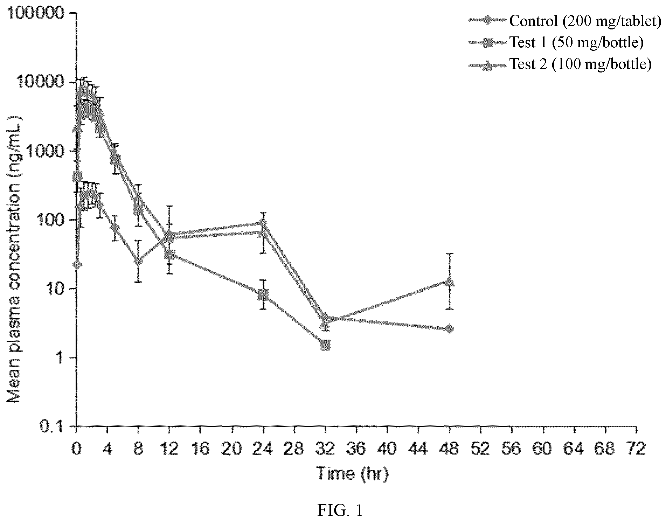

FIG. 1 is a graph of the mean drug concentration-time curves of pazopanib after oral administration of the test and control formulations in Example 7 to beagle dogs (N = 3).

### DETAILED DESCRIPTION

The embodiments of the present disclosure will be further described in detail with reference to the following specific examples. It will be appreciated that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products, or can be prepared by using known methods.

### Examples 1 to 10

The formulas for the examples are shown in Tables 1-1 and 1-2.

**Table 1-1**

| Raw and auxiliary materials | Effect | Example 1 | Example 2 | Example 3 | Example 4 | Example 5 |
|---|---|---|---|---|---|---|
| Pazopanib hydrochloride | Active ingredient | 150mg (7.5mg/ml) | 150mg (7.5mg/ml) | 150mg (7.5mg/ml) | 200mg (10mg/ml) | 200mg (10mg/ml) |
| Soluplus | Carrier material | 400mg (20mg/ml) | 200mg (10mg/ml) | / | 400mg (20mg/ml) | 20mg (1mg/ml) |
| TPGS | Carrier material | / | 200mg (10mg/ml) | 200mg (10mg/ml) | / | / |
| PVP | Carrier material | 100mg (5mg/ml) | / | / | 100mg (5mg/ml) | / |
| HPMC | Carrier material | / | / | 50mg (2.5mg/ml) | / | / |
| Ethanol | Solvent | 2ml (10%v/v) | 4ml (20%v/v) | 3ml (15%v/v) | 2ml (10%v/v) | 4ml (20%v/v) |
| Propylene glycol | Solvent | / | 1ml (5%v/v) | 3ml (15%v/v) | / | 3ml (15%v/v) |
| Glycerol | Solvent | 4ml (20%v/v) | 5ml (25%v/v) | 6ml (30%v/v) | 12ml (60%v/v) | 8ml (40%v/v) |
| Stevioside | Sweetener | / | / | / | / | 100mg (5mg/ml) |
| Essence | Sweetener | 50mg (2.5mg/ml) | 50mg (2.5mg/ml) | 50mg (2.5mg/ml) | 50mg (2.5mg/ml) | / |
| Water | Solvent | 14ml (70%v/v) | 10ml (50%v/v) | 8ml (40%v/v) | 6ml (30%v/v) | 5ml (25%v/v) |

**Table 1-2**

| Raw and auxiliary materials | Effect | Example 6 | Example 7 | Example 8 | Example 9 | Example 10 |
|---|---|---|---|---|---|---|
| Pazopanib hydrochloride | Active ingredient | 200mg (10mg/ml) | 200mg (10mg/ml) | 250mg (12.5mg/ml) | 250mg (12.5mg/ml) | 250mg (12.5mg/ml) |
| Soluplus | Carrier material | 100mg (5mg/ml) | 400mg (20mg/ml) | 800mg (40mg/ml) | 1000mg (50mg/ml) | 2000mg (100mg/ml) |
| TPGS | Carrier material | / | / | / | / | / |
| PVP | Carrier material | / | / | / | / | / |
| HPMC | Carrier material | / | / | / | / | / |
| Ethanol | Solvent | 5ml (25%v/v) | 4ml (20%v/v) | 5ml (25%v/v) | 6ml (30%v/v) | 5ml (25%v/v) |
| Propylene glycol | Solvent | / | 3ml (15%v/v) | / | 2ml (10%v/v) | 3ml (15%v/v) |
| Glycerol | Solvent | 10ml (50%v/v) | 10ml (50%v/v) | 12ml (60%v/v) | 10ml (50%v/v) | 10ml (50%v/v) |
| Stevioside | Sweetener | 100mg (5mg/ml) | 100mg (5mg/ml) | / | / | / |
| Essence | Sweetener | / | / | / | / | / |
| Water | Solvent | 5ml (25%v/v) | 3ml (15%v/v) | 3ml (15%v/v) | 2ml (10%v/v) | 2ml (10%v/v) |

The preparation process comprises the following steps: according to the formulas described above,
1. dissolving a polymeric carrier and a sweetener in a proper amount of water, and uniformly stirring to obtain a clarified and transparent solution A;
2. adding the organic solution into the solution A obtained in the step 1, and continuously stirring to obtain a clarified and transparent solution B; and
3. adding the active drug into the solution B obtained in the step 2, continuously stirring, and filtering through a filter membrane of 0.45 µm to obtain the pazopanib pharmaceutical composition.

### Stability Investigation

The pazopanib oral solution was prepared according to the pharmaceutical formulation formulas listed in the ten examples described above, the pH value of the solution was adjusted to be 3.0-4.5, and the solution was packaged by adopting a brown bottle.

Investigation items were as follows: color, clarity, appearance, content, and degradation product, and the results are shown in Table 2.

**Table 2. Pazopanib oral solution investigation results**

| Oral solution | Investigation item | | | | |
|---|---|---|---|---|---|
| | Color | Clarity | Appearance | Content (HPLC, %) | Total impurities (HPLC, %) |
| Example 1 | Colorless | Clarified | Colorless and clarified liquid with aromatic odor | 100.5 | 0.12 |
| Example 2 | Colorless | Clarified | Colorless and clarified liquid with aromatic odor | 98.5 | 0.17 |
| Example 3 | Colorless | Clarified | Colorless and clarified liquid with aromatic odor | 99.4 | 0.15 |
| Example 4 | Colorless | Clarified | Colorless and clarified liquid with aromatic odor | 101.0 | 0.20 |
| Example 5 | Colorless | Clarified | Colorless and clarified liquid with aromatic odor | 99.6 | 0.11 |
| Example 6 | Colorless | Clarified | Colorless and clarified liquid with aromatic odor | 100.8 | 0.16 |
| Example 7 | Colorless | Clarified | Colorless and clarified liquid with aromatic odor | 98.5 | 0.15 |
| Example 8 | Colorless | Clarified | Colorless and clarified liquid | 101.2 | 0.18 |
| Example 9 | Colorless | Clarified | Colorless and clarified liquid | 98.9 | 0.11 |
| Example 10 | Colorless | Clarified | Colorless and clarified liquid | 100.4 | 0.13 |

The present disclosure prepared the pazopanib oral solution as in Example 7, and carried out influencing factor testing including high-temperature (60 °C) testing, high-humidity (90% RH ± 5% RH) testing, illumination (total illumination: 1.2 × 10^6 Lux hr, total ultraviolet irradiance 200 W•hr/m²) testing and accelerated testing (40 °C/75% RH), and the results are shown in Table 3 below.

**Table 3. Pazopanib oral solution stability investigation results**

| **Investigation item** | | **Day 0** | **High temperature 60 °C** | | | | **High humidity 90% RH ± 5% RH** | | **Illumination (total illumination: 1.2 × 10^6 Lux hr, total ultraviolet radiation 200 W•hr/m²)** | | **Accelerated for 1 month** | |
|---|---|---|---|---|---|---|---|---|---|---|---|---|
| | | | **Day 10** | | **Day 30** | | **Day 10** | | | | | |
| | | | **Put upright** | **Put upside down** | **Put upright** | **Put upside down** | **Put upright** | **Put upside down** | **Put upright** | **Put upside down** | **Put upright** | **Put upside down** |
| Appearance | Appearance | Colorless and clear liquid | Light yellow and clear liquid | Light yellow and clear liquid | Light yellow and clear liquid | Light yellow and clear liquid | Colorless and clear liquid | Colorless and clear liquid | Colorless and clear liquid | Colorless and clear liquid | Light yellow and clear liquid | Light yellow and clear liquid |
| Identification | HPLC | In accordance with the standard | / | | | | | | | | | |
| | UV | In accordance with the standard | | | | | | | | | | |
| Inspection | pH value | 4.47 | 4.38 | 4.36 | 4.36 | 4.33 | 4.32 | 4.34 | 4.34 | 4.38 | 4.40 | 4.32 |
| | Clarity | Clarified solution | Clarified solution | Clarified solution | Clarified solution | Clarified solution | Clarified solution | Clarified solution | Clarified solution | Clarified solution | Clarified solution | Clarified solution |
| | Related substances | BRL | 0.09% | 0.07% | 0.11% | 0.12% | BRL | BRL | BRL | BRL | 0.07% | 0.06% |
| | | 0.00% | 0.16% | 0.18% | 0.29% | 0.26% | 0.00% | 0.00% | 0.00% | 0.00% | 0.24% | 0.21% |
| | Amount of ethanol | 18.9% | 18.7% | 18.1% | 19.0% | 19.2% | 19.1% | 17.9% | 18.7% | 18.8% | 18.6% | 19.0% |
| Others | Deliverable volume | In accordance with the standard | / | | | | | | | | | |
| Content measurement (%) | ChP | 100.6 | 100.3 | 100.4 | 98.2 | 96.8 | 101.7 | 101.3 | 100.1 | 99.9 | 97.6 | 97.5 |
| Relative density | / | 1.119 | 1.123 | 1.123 | 1.120 | 1.123 | 1.124 | 1.122 | 1.109 | 1.109 | 1.23 | 1.22 |

The stability results show that the stability of Example 7 was good.

### Animal PK assay

As a test formulation, the pazopanib oral solution was prepared as in Example 7, with a commercially available pazopanib tablet (VOTRIENT, 200 mg/tablet) as a control formulation. Beagle dogs were orally administered with the pazopanib oral solution at a dose of 50 mg/dog/day and 100 mg/dog/day, respectively, beagle dogs were orally administered with VOTRIENT at a dose of 200 mg/dog/day, and pharmacokinetic properties of the control formulation and the test formulation at different doses in beagle dogs were examined. The pharmacokinetic results are shown in Table 4, and PK curves are shown in FIG. 1.

Test results show that the exposure amount of the API could be still significantly improved on the premise that the dosage of the API was 2 times and 4 times lower than that of a control dose in Example 7, and the oral bioavailability was significantly improved.

**Table 4. Pharmacokinetic examination results of pazopanib oral solutions and tablets in dogs**

| **Time (hr)** | **Concentration (ng/mL)** | | |
|---|---|---|---|
| | **Control formulation VOTRIENT- 200 mg** | **Test formulation Pazopanib oral solution-50 mg** | **Test formulation Pazopanib oral solution-100 mg** |
| 0.167 | 21.9 | 421 | 2193 |
| 0.5 | 152 | 3373 | 7293 |
| 1 | 223 | 4233 | 8537 |
| 1.5 | 226 | 4117 | 7103 |
| 2 | 243 | 3677 | 6630 |
| 2.5 | 224 | 3147 | 5640 |
| 3 | 162 | 2127 | 3677 |
| 5 | 75.1 | 737 | 903 |
| 8 | 24.6 | 139 | 213 |
| 12 | 59.9 | 31.5 | 54.0 |
| 24 | 88.0 | 8.19 | 64.9 |
| 32 | 3.76 | 1.51 | 3.08 |
| 48 | 2.53 | BQL | 12.7 |
| 72 | BQL | BQL | BQL |
| Tₘₐₓ (hr) | 5.33±5.80 | 1.17±0.764 | 1.00±0.00 |
| Cₘₐₓ (mg/mL) | 257±96.3 | 4550±1100 | 8537±3403 |
| T_{1/2} (hr) | 2.76±2.22 | 3.47±1.38 | 5.26±0.838 |
| AUCₗₐₛₜ (hr*ng/mL) | 1789±1111 | 14416±4258 | 26359±10854 |
| AUC_{INF} (hr*ng/mL) | 1800±1118 | 14425±4259 | 26470±10702 |

As shown above, the pazopanib oral solution of the present disclosure has good stability, can improve the mouthfeel of the formulation, and has remarkably improved bioavailability compared with the control formulation.

The above examples illustrate the embodiments of the present disclosure. However, the present disclosure is not limited to the embodiments described above. Any modification, equivalent, improvement, and the like made without departing from the spirit and principle of the present disclosure shall fall within the protection scope of the present disclosure.

## Claims

1. A pazopanib oral pharmaceutical composition, comprising: an active drug, a polymeric carrier, an organic solvent and/or water, wherein the active drug is 5-[[4-[(2,3-dimethyl-2H-indazol-6-yl)methylamino]pyrimidin-2-yl]amino]-2-methylbenzenesulfonamide represented by formula I or a salt thereof;

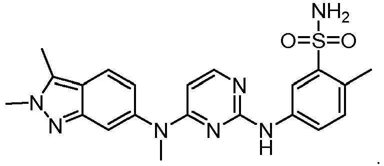

2. The pazopanib oral pharmaceutical composition according to claim 1, wherein a pH value of the pazopanib oral pharmaceutical composition is 3.0-4.5;
preferably, the polymeric carrier comprises one or more of polyvinyl caprolactam-polyvinyl acetate-polyethylene glycol graft copolymer, poloxamer, polyethylene glycol vitamin E succinate, 15-hydroxystearic acid polyethylene glycol ester, polyoxyethylene 40 hydrogenated castor oil, polyoxyl 35 hydrogenated castor oil, povidone, crospovidone, hydroxypropyl methylcellulose, hydroxypropylcellulose, polyvinyl alcohol, tween 80, polyvinylpyrrolidone, and polyethylene glycol.

3. The pazopanib oral pharmaceutical composition according to claim 1 or 2, wherein the active drug is 5-[[4-[(2,3-dimethyl-2H-indazol-6-yl)methylamino]pyrimidin-2-yl]amino]-2-methylbenzenesulfonamide hydrochloride;
and/or,
the weight ratio of the active drug to the polymeric carrier is 1: 10-10: 1;
and/or,
the concentration of the active drug is 5-20 mg/mL.

4. The pazopanib oral pharmaceutical composition according to any one of claims 1-3, wherein the organic solvent comprises one or more of ethanol, propylene glycol, glycerol and polyethylene glycol-300, polyethylene glycol-400 and polyethylene glycol-600;
and/or,
the volume ratio of the organic solvent to the pazopanib pharmaceutical composition is 0.3-0.9, and the volume ratio refers to the ratio of the volume of the organic solvent to the total volume of the pazopanib pharmaceutical composition.

5. The pazopanib oral pharmaceutical composition according to any one of claims 1 to 4, wherein the pazopanib oral pharmaceutical composition further comprises a sweetener;
preferably, the sweetener comprises one or more of aspartame, sucralose, fructose, sucrose, stevioside, glycyrrhizin, essence, spices, saccharin and sodium saccharin;
preferably, the mass ratio of the sweetener to the active drug is 1:5-5:1.

6. The pazopanib oral pharmaceutical composition according to any one of claims 1-5, wherein the pazopanib oral pharmaceutical composition is selected from any one of the following formulas:
Formula 1: 7.5 mg/mL pazopanib hydrochloride, 20 mg/mL soluplus, 5 mg/mL PVP, 10% v/v of ethanol, 20% v/v of glycerol, 2.5 mg/mL essence, and 70% v/v of water;
Formula 2: 7.5 mg/mL pazopanib hydrochloride, 10 mg/mL soluplus, 10 mg/mL TPGS, 20% v/v of ethanol, 5% v/v of propylene glycol, 25% v/v of glycerol, 2.5 mg/mL essence, and 50% v/v of water;
Formula 3: 7.5 mg/mL pazopanib hydrochloride, 10 mg/mL TPGS, 2.5 mg/mL HPMC, 15% v/v of ethanol, 15% v/v of propylene glycol, 30% v/v of glycerol, 2.5 mg/mL essence, and 40% v/v of water;
Formula 4: 10 mg/mL pazopanib hydrochloride, 20 mg/mL soluplus, 5 mg/mL PVP, 10% v/v of ethanol, 60% v/v of glycerol, 2.5 mg/mL essence, and 30% v/v of water;
Formula 5: 10 mg/mL pazopanib hydrochloride, 1 mg/mL soluplus, 20% v/v of ethanol, 15% v/v of propylene glycol, 40% v/v of glycerol, 5 mg/mL stevioside, and 25% v/v of water;
Formula 6: 10 mg/mL pazopanib hydrochloride, 5 mg/mL soluplus, 25% v/v of ethanol, 50% v/v of glycerol, 5 mg/mL stevioside, and 25% v/v of water;
Formula 7: 10 mg/mL pazopanib hydrochloride, 20 mg/mL soluplus, 20% v/v of ethanol, 15% v/v of propylene glycol, 50% v/v of glycerol, 5 mg/mL stevioside, and 15% v/v of water;
Formula 8: 12.5 mg/mL pazopanib hydrochloride, 40 mg/mL soluplus, 25% v/v of ethanol, 60% v/v of glycerol, and 15% v/v of water;
Formula 9: 12.5 mg/mL pazopanib hydrochloride, 50 mg/mL soluplus, 30% v/v of ethanol, 10% v/v of propylene glycol, 50% v/v of glycerol, and 10% v/v of water; and
Formula 10: 12.5 mg/mL pazopanib hydrochloride, 100 mg/mL soluplus, 25% v/v of ethanol, 15% v/v of propylene glycol, 50% v/v of glycerol, and 10% v/v of water.

7. A method for preparing the pazopanib oral pharmaceutical composition according to any one of claims 1-6, comprising the following steps:
(1) mixing the polymeric carrier and the sweetener with water to form a solution A;
(2) mixing the organic solvent with the solution A obtained in the step (1) to obtain a solution B; and
(3) mixing the active drug with the solution B obtained in the step (2), stirring and filtering to obtain the pazopanib pharmaceutical composition.

8. Use of the pazopanib oral pharmaceutical composition according to any one of claims 1-6 for manufacturing of a medicament or a pharmaceutical formulation for treating and/or preventing a tumor; wherein
preferably, the tumor is renal cancer or soft tissue sarcoma.

9. A pharmaceutical formulation comprising the pazopanib oral pharmaceutical composition according to any one of claims 1-6.

10. The pharmaceutical formulation according to claim 9, wherein the pharmaceutical formulation is an oral liquid.
